# EUROPEAN PATENT SPECIFICATION

(11) **EP 0 443 789 B1**
(45) Date of publication and mention of the grant of the patent: **21.06.1995**
(21) Application number: 91301247.2
(22) Date of filing: 15.02.1991
(51) Int. Cl.: A23L 1/30

(54) **Use of refined pyrodextrin hydrolysate**
Verwendung von raffinierten Pyrodextrinhydrolysat
Utilisation d'un hydrolisat raffiné de pyrodextrin

(30) Priority: 20.02.1990 JP 40384/90
(43) Date of publication of application: 28.08.1991
(73) Proprietor: MATSUTANI CHEMICAL INDUSTRIES CO. LTD., Itami-shi, Hyogo-ken (JP)
(72) Inventor: Ohkuma, Kazuhiro, Sanda-shi, Hyogo-ken (JP); Wakabayashi, Shigeru, Sanda-shi, Hyogo-ken (JP); Mochizuki, Yoshimi, Itami-shi, Hyogo-ken (JP); Satouchi, Mitsuko, Takarazuka-shi, Hyogo-ken (JP)
(74) Representative: Low, Peter John

(56) References cited:
- DATABASE BIOSIS AN80:129661, Biochemical Abstracts, Philadelphia, US, M.J. ALBRINK et al.: "Effect of high fiber and low fiber diets on plasma lipids and isulin" & Am. J. Clin. Nutr. 1979, vol.32, no. 7, pages 1486-1491
- CHEMICAL ABSTRACTS vol. 109, no. 12, 19 September 1988, page 99, abstract no. 95048c, Columbus, Ohio, US; C.T. YANG et al.: "Manufacture of pyrodextrins from rice. I. Optimal conditions for the methods of pyrodextrinization" & Chung-Kuo Nung Yeh Hua Hsueh Hui Chih 1988, vol. 26, no. 1, pp. 63-68
- CHEMICAL ABSTRACTS vol. 111, no. 17, 23 October 1989, page 607, abstract no. 152572m, Columbus, Ohio, US; F.R.J. BORNET et al.:"Insulin and glycemic responses in healthy humans to native starches processed in different ways: correlation with in vitro alpha-amylase hydrolysis" & Am. J. Clin. Nutr. 1989, vol. 50, no. 2,pages 315-323
- CHEMICAL ABSTRACTS vol. 91, no. 15, 8 October 1979, abstract no. 122401f, Columbus, Ohio, US; N.N. GODBOLE: "Characteristics of different pyrodextrins" & J. Diabetic Assoc. India 1979,vol. 19, no. 1, pages 9-11

## Description

### BACKGROUND OF THE INVENTION

### 1. Field of the invention:

The present invention relates to a use of a refined pyrodextrin hydrolysate for reducing insulin secretion without negatively affecting/influencing blood glucose levels in an animal.

### 2. Description of Prior Art:

The increase of blood glucose after eating a meal results in secretion of insulin. Because of recent progress in food refining processes and people's preferences for their favourite taste, it is a tendency for the people of European and American countries to consume a good deal of sugar and fat. Consequently, the rate of occurrence of non-infectious diseases such as obesity, diabetes and arteriosclerosis is now extremely high due to this excessive intake of sugar and fat and the resultant excessive secretion of insulin that occurs. Also, in Japan with the recent westernisation of the diet there has been a trend for the rate of the occurrence of the abovementioned diseases to sharply increase.

Hitherto, several materials that act on the autonomous nervous system have been well known. For example, as a stimulant for α-receptors in sympathetic nerves such materials as epinephirine and norepinephirine have been well known for performing the function of inhibiting insulin secretion and as an interceptor for β-receptor in the sympathetic nerve, such materials as propanol and dopaserotonin have been well known for performing the same function.

In addition, sugar metabolism inhibitors such as 2-desoxyglucose and mannoheptulose, somatostatin and Ca²⁺ antagonists also perform the function of inhibiting insulin secretion.

A problem, however, exists in that most of these materials, belong to a group of powerful medicines which are specified in the Japanese "Drugs Cosmetics and Medical Instruments Law" as being prohibited food additives.

Furthermore, it has been generally said that water-soluble dietary fibers, typically represented by guar gum and pectin are useful for reducing insulin secretion because of their inhibition of increases in blood glucose. It is, however, also said that these fibers have the disadvantage of having a negative affect on the adsorption of useful metals due to their high viscosity. Such high viscosity brings about a further disadvantage of making it difficult to consume or ingest them in a large quantity and, as a result, uses of these fibers are quite limited.

As noted above, westernization and diversification of diet has increasingly taken place in Japan. In particular, with the improvement in the refining techniques of food, occurrence of non-infectious diseases such as obesity, diabetes, and arteriosclerosis has been increasing due to an excessive intake of sugars and fat, in particular, due to disorders in sugar metabolism caused thereby. In the prior art, materials performing an effect of lowering blood glucose such as various polysacchardies including guar gum, a devil's tongue mannan, and alpha -glucosidase inhibitor have been conventionally employed for the purpose of prevention and remedy of the mentioned diseases. On the other hand, it is known to those skilled in the art that the increment of insulin secretion is in proportion to the amount of increase in blood glucose value per unit time in the early stages, while being in proportion to the total amount of sugar content ingested at the later stage. Furthermore, the blood glucose value after ingestion, particularly the highest blood sugar value is not always correlated with the amount of sugar content ingested. Furthermore, it has been recognised that the change in the blood glucose value is one of the important factors in determining the start and finish of a meal and is deeply related to the adjustment of every meal amount in a rather short time.

When considering the aforesaid knowledge, a reduction in insulin secretion without having an affect on the blood glucose level, in other words, without disturbing the physiology of eating behaviour, would be very significant not only for the prevention of obesity, inhibition of the transition from the pre-stage of diabetes to obvious diabetes, but also for the inhibition of fat deposition caused by insulin in arteriosclerosis. Moreover, it is desired to exhibit certain useful effects for the prevention from diseases in general by way of reducing the harmful effects brought about by insulin. From the mentioned quite novel point of view, the inventors have come to conceive of an idea of developing a novel material capable of performing the advantage of reducing insulin secretion without negatively affecting the blood glucose level in both human and animal bodies.

On the basis of such a new concept, the inventors have further come to an attempt of materialising the novel idea in order to assure the advantage of reducing the mentioned insulin secretion utilising pyrodextrin which has hardly been considered as a food material, thus developing a new food composite performing the mentioned effect.

In Chemical Abstracts vol. 91, no. 15, 1979, abstract no. 122401f pyrodextrins obtained by pyrolysis (201°C for 15 to 20 minutes) are mentioned and the said powderised products are used in diabetic diets. Also in Chemical Abstracts vol. 111, no. 17, 1989, abstract no. 152572m, starches cooked and cooled after a preliminary extrusion cooking and in vitro alpha-amylase hydrolysis are disclosed. However, none of these documents disclose any process of refining the pyrodextrin.

### SUMMARY OF THE INVENTION

Accordingly, an object of the present invention is to materialise a new idea as mentioned above, in other words, to develop a new food composite from pyrodextrin on the basis of the mentioned new idea, the new food composite being capable of performing the action of reducing insulin secretion without an undesirable influence on the blood glucose level.

The foregoing object of the invention is accomplished by employing a redefined product of pyrodextrin that can be obtained through a process wherein starch or starch hydrolysate is decomposed by heating in the presence of an acid or without an acid, the mentioned refined pyrodextrin serving as a main composition of food composites performing an advantage of reducing insulin secretion.

As mentioned above, the invention is based on a novel concept that the refined pyrodextrin obtained through the process of decomposing at least one of starch or starch hydrolysate exhibits an extremely remarkable action of reducing insulin secretion without affecting the blood glucose level.

According to the present invention there is provided the use of a refined pyrodextrin hydrolysate for the preparation of a food composite for reducing insulin secretion without negatively affecting/influencing blood glucose levels in an animal, wherein said refined pyrodextrin hydrolysate is obtainable by heating starch in the presence of a mineral acid and in the presence of water of not more than about 5% based on the weight of the starch to prepare pyrodextrin, hydrolysing the pyrodextrin with alpha-amylase and refining the hydrolysed pyrodextrin.

The term animal is intended to include all animate insulin secreting beings including humans.

Preferably, the refined pyrodextrin hydrolysate is structurally composed of glucose having linkages of 1→2 and 1→3 bonds as well as 1→4 and 1→6 bonds, and a part of their reducing end groups is of 1→6 anhydroglucose.

Preferably, the said refined pyrodextrin hydrolysate is obtained by heating said starch in the presence of hydrochloric acid in an amount of 0.01 to 0.1% by weight based on the weight of said starch at 150°C to 220°C for 1 to 5 hours to prepare pyrodextrin, dissolving the pyrodextrin in water in an amount of 30 to 50% by weight based on the weight of the solution, adjusting the pH of the solution to pH 5.5 to 6.5, treating the solution with alpha-amylase in an amount of 0.05 to 0.2% by weight based on the weight of pyrodextrin at 85°C to 100°C for 0.5 to 2 hours, and refining the hydrolysed pyrodextrin.

According to a second aspect of the present invention there is provided the use of a refined pyrodextrin hydrolysate for the preparation of a food composite for reducing insulin secretion without negatively affecting/influencing blood glucose levels in an animal, wherein said refined pyrodextrin hydrolysate is obtainable by heating starch in the presence of a mineral acid and in the presence of water of not more than about 5% based on the weight of the starch to prepare pyrodextrin, hydrolysing the pyrodextrin with alpha-amylase and then with glucoamylase, and subjecting the hydrolysed dextrin to ion exchange resin chromatography to refine the hydrolysed pyrodextrin.

Preferably, the said refined pyrodextrin hydrolysate is structurally composed of glucose having linkages of 1→2 and 1→3 bonds as well as 1→4 and 1→6 bonds, and a part of their reducing end groups is of 1→6 anhydroglucose.

Preferably, the said refined pyrodextrin hydrolysate is obtained by heating said starch in the presence of hydrochloric acid in an amount of 0.01 to 0.1% by weight based on the weight of said starch at 150°C to 220°C for 1 to 5 hours to prepare pyrodextrin, dissolving the pyrodextrin in water in an amount of 30 to 50% by weight based on the weight of the solution, adjusting the pH of the solution to pH 5.5 to 6.5, treating the solution with alpha-amylase in an amount of 0.05 to 0.2% by weight based on the weight of the pyrodextrin at 85°C to 100°C for 0.5 to 2 hours, treating the solution with glucoamylase in an amount of 0.05 to 0.2% by weight was based on the weight of the pyrodextrin at about 55°C for 24 to 48 hours, refining the hydrolysed pyrodextrin and subjecting the hydrolysed and refined pyrodextrin to ion exchange resin chromatography to seprarate said pyrodextrin hydrolysate.

Preferably, the said ion exchange resin is an alkaline metal or alkaline earth metal type of strongly acidic ion exchange resin.

Described now is a process for preparing pyrodextrin in accordance with the invention.

As for a raw starch, that is, a starch to be employed in embodying the invention, a wide range of starches such as potato, corn and cassaba can be utilised. It is also preferable to employ these starches in the form of a processed food commercially available in the market. In this case, enumerated as processed starches are, for example, soluble starch, esterified starch, etherised starch, cross-linked starch and, preferably, starch phosphate and hydroxypropyl starch.

These raw materials are in accordance with the invention decomposed by heating, preferably, under normal pressure. The decomposition by heating is acheived by heating the materials at a temperature from about 130°C to 220°C for 1 to 5 hours. The pressure during heating may be normal without the necessity for putting the materials under vacuum or pressure. At the heating step, it is preferable to add an acid as a catalyst for the decomposition by heating. The acid catalyst may be a mineral acid such as sulfuric acid, hydrochloric acid or nitric acid and in particular, hydrochloric acid is preferable and is preferably added in an amount of several % by weight to the materials to have a concentration of 1% by weight. The acid should be added evenly, being, preferably, well mixed by spraying. The mixture is then preferably dried up preliminarily at a temperature from about 100°C to 120°C so as to reduce the moisture to not more than 5%.

The dextrin in accordance with the invention is obtained in the mentioned process, that is, pyrodextrin is then subject to refining. The refining process is now described hereunder:

At least one treatment of following (a) and (b) are employed:
(a) After a hydrolysis with α-amylase, or after hydrolysis with glucoamylase following the hydrolysis with α-amylase, the solution is refined through known processes of filtration, decolorisation, and deionisation.
(b) After completion of the treatment (a), a further treatment separates the dextrin fraction using chromatography with ion-exchange resins.

Further description on the treatments (a) and (b) are given in detail as follows:
In the treatment (a), pyrodextrins are dissolved in water to obtain a solution of 30% to 50% by weight, and then neutralised to pH5.5 to 6.5, preferably to pH5.8 α-amylase, 0.05% to 0.2% by weight (available in the market, either the one originated from mould or the one from bacteria may be applied) based on the pyrodextrin is then neutralised to pH5.5 to 6.5, preferably to pH5.8 α-amylase, 0.05% to 0.2% by weight (available in the market, either the one originated from mould or the one from bacteria may be applied) based on the pyrodextrin is added to the solution, and then at the reaction temperature of said amylase in the range from about 85°C to 100°C, the solution is held at this temperature for 30 minutes to 2 hours. Subsequently, the temperature of the solution is raised up to 120°C to complete the reaction of α-amylase. Thereafter, the temperature of the solution is decreased to about 55°C, and the solution is adjusted to about pH5.5, then glucoamylase, 0.05% to 0.2% by weight, (popularly used) based on the original dextrin is added. The solution is kept at a temperature that allows its reaction for 24 to 48 hours. This reaction aims at decomposition of small molecules such as oligosaccharides into glucose. Following this step, temperature of the solution is raised up to, for example, about 80°C to complete the action of glucoamylase.

On the other hand, in the treatment (b), chromatographic separation by ion-exchanger resin is carried out. In this treatment, strongly acidic ion exchanger resins sold widely on the market can be employed.

Preferably, by way of examples Amberlite IR-116, IR-118, IR-120B, XT-1022E, XT-471F (all manufactured by Organo), Diaion SK-1B, SK-102, SK-104, SK-106, SK-110, SK-112, SK-116, FR-01 (all manufactured by Mitsubishi Chemicals), and XFS-43281.00, XFS-43280.00, XFS-43279.00, XFS-43278.00 (all manufactured by Daw Chemicals) may be used.

These resins are preferably dealt with as alkaline metal type or alkaline earth metal type before their uses. It is preferable to adjust the rate of flow for each according to the resin to be used. The rate of flow is preferably in the range of SV = 0.1 to 0.6. A rate of flow out of the above range tends to deteriorate the workability and separation of the process. The temperature at the time of running the fluid is preferably in the range from 20°C to 70°C, and a temperature below this range will deteriorate the separation and raise the viscosity of the fluid, thereby giving a negative influence on the fluid, while a temperature exceeding this range will cause the fluid to be tanned and deteriorate other quality characteristics.

When observing carefully the dextrin refined from the mentioned pyrodextrin, it was recognized that their linkages were not only 1→4 and 1→6 bonds with glucose as structural sugar, but also 1→2 and 1→3 bonds. Further, a part of the reducing end group is of 1-6-anhydroglucose.

Viscosity of this pyrodextrin is rather low, i.e., about 10mPa.s(cps) (30%, 30°C), and it tastes slightly sweet and is odourless, with the number of 1→2 and 1→3 bonds below about 10%. The pyrodextrin is, therefore, easily employed as a material for various beverages and processed foods and, furthermore, it is as safe to be eaten as maltodextrin since its raw material is starch.

In this manner, the pyrodextrin serving as the abvoe food material in accordance with the invention can be widely used as a material for various foods, and its uses extend to any food so far as it is used as a material for food. Typical foods in this sense are, for example, beverages, desserts and candies.

Other objects, features and advantages of the invention will become apparent in the course of the following description accompanied by the drawings.

### BRIEF DESCRIPTION OF THE DRAWINGS

Figure 1 is a graph showing the results of measuring blood glucose values and insulin values;
Figure 2 is a graph showing the results of measuring blood glucose values; and
Figure 3 is a graph showing the results of measuring insulin values.

### DESCRIPTION OF THE PREFERRED EMBODIMENTS

Several examples in accordance with the present invention are hereinafter described in more detail.

### Example 1:

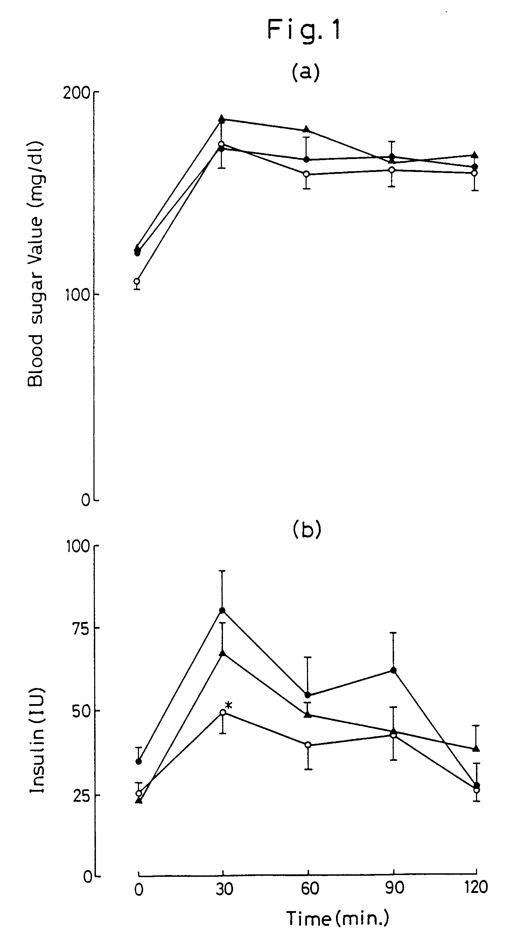

8-week-old male rats of SD descent weighing 234g to 244g were employed as samples. After subjecting the rats to a fast for about 20 hours, three kinds of solution, namely, a solution of glucose alone (1.5g/kg weight), a solution of glucose (1.5g/kg weight) and PF (0.6g/kg weight), a solution of glucose (1.5g/kg weight) and PF-C (0.6g/kg weight) were given orally to them, blood samples were collected from the samples periodically, and blood glucose levels thereof were measured by an enzyme method, with insulin values being measured by an RIA method. Each administrative test was carried out at one-week intervals. The results are shown in Figure 1, wherein (a) shows the measured values of blood glucose values, and (b) shows the measured values of insulin. In the drawing, the mark ● indicates the case with a solution of glucose alone, the mark △ indicates the case with a solution of glucose and PF, and the mark ○ indicates the case with a solution of glucose and PF-C. The mentioned PF-C and PF are defined as follows:
- PF-C;: a substance obtained by a process wherein pyrodextrin is hydrolysed with α-amylase and glucoamylase, then the glucose produced thereby is separated by column chormatography method.
- PF:: a substance obtained by a process wherein pyrodextrin is hydrolyzed with α-amylase, then refined by decolorisation with activated charcoal and dealt with by ion-exchanger resin.

From the results shown in Figure 1, total sum (ΣBG, ΣIRI and ΣIRI/ΣBG) of the blood glucose values and insulin values at each measurement point were calculated respectively as shown in Table 1.

**TABLE 1**

| The Results of GTT (mean ± SD) on Healthy Rats (SD descent, 8-week old, male) | | | |
|---|---|---|---|
| | Load with glucose alone | Load with glucose + PF-C | Load with glucose + PF |
| n | 7 | 7 | 4 |
| ΣBG | 784.2 ± 68.8 | 754.1 ± 49.8 | 821.5 ± 41.2 |
| | | NS | NS |
| ΣIRI | 254.5 ± 49.8 | 177.3 ± 49.8 | 194.2 ± 36.0 |
| | | p < 0.02 | p < 0.10 |
| ΣIRI/ΣBG | 0.33 ± 0.07 | 0.23 ± 0.05 | 0.24 ± 0.05 |
| | | p < 0.02 | p < 0.05 |

As is understood from Figure 1 and Table 1, the blood glucose values and ΣBG at each measurement point exhibited no difference to be noted between the load groups of glucose alone and glucose + PF-C. On the other hand, the insulin values in the administrative group of glucose + PF and glucose + PF-C were conspicuously lower than those in the administrative group of glucose alone. Furthermore, significant decreases in ΣIRI and IRI/ΣBG were recognised.

### Example 2:

Six healthy adult men were employed as samples.

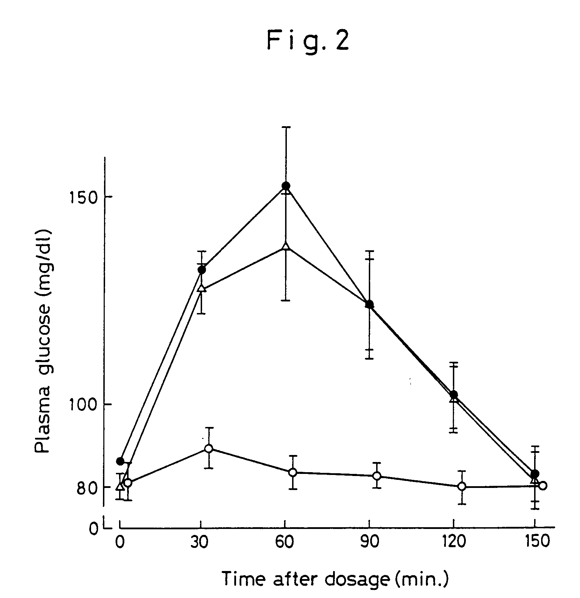

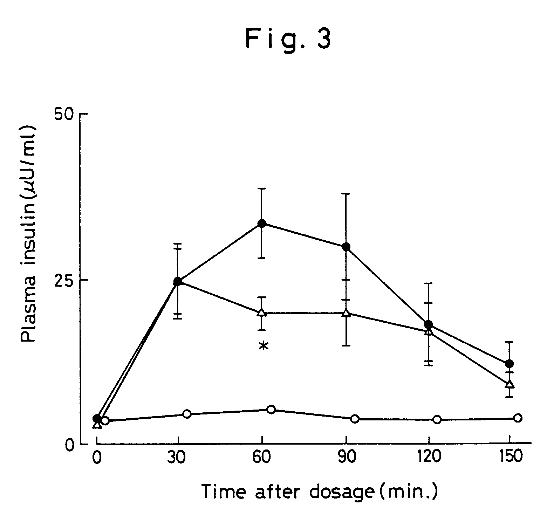

After being subject to a fast for 24 hours, 50g of glucose alone, 50g of glucose and 20g of PF-C, or 20g of PF-C alone were given orally to them respectively, then blood samples were collected from the adult men samples periodically, and the blood glucose levels thereof were measured and insulin values were measured by an RIA method. The results of the measurements are shown in Figure 2 for the blood glucose values and in Figure 3 for the insulin values. ΣBG, ΣIRI and ΣIRI/ΣBG are shown in Table 2.

**TABLE 2**

| The Results of GTT (mean ± SD) on Healthy Adult Men | | |
|---|---|---|
| | Administration with glucose alone | Administration with glucose + PF-C |
| n | 6 | 6 |
| ΣBG | 658.8 ± 100.3 | 648.6 ± 107.8 |
| | | NS |
| ΣIRI | 136.5 ± 48.3 | 90.5 ± 17.8 |
| | | NS (p < 0.10) |
| ΣIRI/ΣBG | 0.20 ± 0.05 | 0.14 ± 0.02 |
| | | NS (p < 0.05) |

In Figures 2 and 3, the mark ● indicates the case of glucose alone, the mark ○ indicates the case of 20g of PF-C and the marks △ indicates the case of 50g of glucose and 20g of PF-C.

As is understood from Figure 2, PF-C did not affect blood glucose values by itself. Also, the increase of blood glucose by the simultaneous dosage of glucose and PF-C did not show any difference from that by the single dosage of glucose.

As is understood from Figure 3, the single dosage of PF-C did not bring about insulin secretion, either.

Further, insulin secretion by the simultaneous dosage of glucose and PF-C did show a significant drop within 60 minutes as compared with that by the single dosage of glucose. Furthermore, as is understood from Table 2, ΣBG did not show any diffrence in both the load group of the single dosage of glucose and the group of the simultaneous dosage of glucose and PF-C, while ΣIRI showed a declining tendency in the latter. In addition, ΣIRI/ΣBG, which was calculated as an index of the change in the insulin amount corresponding to the change in blood glucose per unit amount, did show a meaningful lowering in the latter.

## Claims

1. Use of refined pyrodextrin hydrolysate for the preparation of a food composite for reducing insulin secretion without negatively affecting/influencing blood glucose levels in an animal, wherein said refined pyrodextrin hydrolysate is obtainable by heating starch in the presence of a mineral acid and in the presence of water of not more than about 5% based on the weight of the starch to prepare pyrodextrin, hydrolysing the pyrodextrin with alpha-amylase and refining the hydrolysed pyrodextrin.

2. The use according to claim 1, wherein said refined pyrodextrin hydrolysate is structurally composed of glucose having linkages of 1→2 and 1→3 bonds aswell as 1→4 and 1→6 bonds, and a part of their reducing end groups is of 1→6 anhydroglucose.

3. The use according to claim 1, wherein said refined pyrodextrin hydrolysate is obtained by heating said starch in the presence of hydrochloric acid in an amount of 0.01 to 0.1% by weight based on the weight of said starch at 150°C to 220°C for 1 to 5 hours to prepare pyrodextrin, dissolving the pyrodextrin in water in an amount of 30 to 50% by weight based on the weight of the solution, adjusting the pH of the solution to pH 5.5 to 6.5, treating the solution with alpha-amylase in an amount of 0.05 to 0.2% by weight based on the weight of the pyrodextrin at 85°C to 100°C for 0.5 to 2 hours, and refining the hydrolysed pyrodextrin.

4. Use of a refined pyrodextrin hydrolysate for the preparation of a food composite for reducing insulin secretion without negatively affecting/influencing blood glucose levels in an animal, wherein said refined pyrodextrin hydrolysate is obtainable by heating starch in the presence of a mineral acid and in the presence of water of not more than about 5% based on the weight of the starching to prepare pyrodextrin, hydrolysing the pyrodextrin with alpha-amylase and then with glucoamylase, and subjecting the hydrolysed dextrin to ion exchange resin chromatography to refine the hydrolysed pyrodextrin.

5. The use according to claim 4, wherein said refined pyrodextrin hydrolysate is structurally composed of glucose having linkages of 1→2 and 1→3 bonds as well as 1→4 and 1→6 bonds, and a part of their reducing end groups is of 1→6 anhydroglucose.

6. The use according to claim 4, wherein said refined pyrodextrin hydrolysate is obtained by heating said starch in the presence of hydrochloric acid in an amount of 0.01 to 0.1% by weight based on the weight of said starch at 150°C to 220°C for 1 to 5 hours to prepare pyrodextrin, dissolving the pyrodextrin in water in an amount of 30 to 50% by weight based on the weight of the solution, adjusting the pH of the solution to pH 5.5 to 6.5, treating the solution with alpha-amylase in an amount of 0.05 to 0.2% by weight based on the weight of the pyrodextrin at 85°C to 100°C for 0.5 to 2 hours, treating the solution with glucoamylase in an amount of 0.05 to 0.2% by weight was based on the weight of the pyrodextrin at about 55°C for 24 to 48 hours, refining the hydrolysed pyrodextrin and subjecting the hydrolysed and refined pyrodextrin to ion exchange resin chromatography to seprarate said pyrodextrin hydrolysate.

7. The use according to claim 6, wherein said ion exchange resin is an alkaline metal or alkaline earth metal type of strongly acidic ion exchange resin.

## Patentansprüche

1. Verwendung eines gereinigten Pyrodextrinhydrolysats zur Herstellung eines Lebensmittelbestandteils zur Reduzierung der Insulinausschüttung ohne negative Auswirkungen/Einflüsse auf den Blutzuckerspiegel bei Tieren, worin das genannte gereinigte Pyrodextrinhydrolysat durch Erhitzen von Stärke in Gegenwart einer Mineralsäure und in Gegenwart von höchstens 5% Wasser, basierend auf dem Gewicht der Stärke zur Herstellung des Pyrodextrins, Hydrolysierung des Pyrodextrins mit α-Amylase und Reinigung des hydrolysierten Pyrodextrins hergestellt werden kann.

2. Verwendung nach Anspruch 1, worin das genannte Pyrodextrinhydrolysat strukturell aus Glucosen mit sowohl 1→2 und 1→3 Bindungen als auch 1→4 und 1→6 Bindungen zusammengesetzt ist und ein Teil ihrer reduzierenden Endgruppen aus 1→6 Anhydroglucose besteht.

3. Verwendung nach Anspruch 1, worin das genannte Pyrodextrinhydrolysat hergestellt wird, indem man zur Herstellung von Pyrodextrin Stärke 1 bis 5 Stunden lang bei 150°C bis 220°C in Gegenwart von 0,01 bis 0,1 Gewichtsprozent Salzsäure, basierend auf dem Gewicht der genannten Stärke, erhitzt, das Pyrodextrin in 30 bis 50 Gewichtsprozent Wasser, basierend auf dem Gewicht der Lösung, löst, die Lösung auf pH 5,5 bis 6,5 einstellt, die Lösung 0,5 bis 2 Stunden lang mit 0,05 bis 0,2 Gewichtsprozent α-Amylase, basierend auf dem Pyrodextringewicht, bei 85°C bis 100°C behandelt und das hydrolysierte Pyrodextrin reinigt.

4. Verwendung eines gereinigten Pyrodextrinhydrolysats zur Herstellung eines Lebensmittelbestandteils zur Reduzierung der Insulinausschüttung ohne negative Auswirkungen/Einflüsse auf den Blutzuckerspiegel bei Tieren, worin das genannte gereinigte Pyrodextrinhydrolysat hergestellt werden kann, indem man zur Herstellung des Pyrodextrins Stärke in Gegenwart einer Mineralsäure und in Gegenwart von höchstens 5% Wasser, basierend auf dem Gewicht der Stärke erhitzt, das Pyrodextrin mit α-Amylase und anschließend Glucoamylase hydrolysiert und das hydrolysierte Pyrodextrin mittels Chromatographie an Ionenaustauschharz reinigt.

5. Verwendung nach Anspruch 4, worin das genannte gereinigte Pyrodextrinhydrolysat strukturell aus Glucosen mit sowohl 1→2 und 1→3 Bindungen als auch 1→4 und 1→6 Bindungen zusammengesetzt ist und ein Teil ihrer reduzierenden Endgruppen aus 1→6 Anhydroglucose besteht.

6. Verwendung nach Anspruch 4, worin das genannte gereinigte Pyrodextrinhydrolysat hergestellt wird, indem man zur Herstellung von Pyrodextrin die genannte Stärke in Gegenwart von 0,01 bis 0,1 Gewichtsprozent Salzsäure, basierend auf dem Gewicht der genannten Stärke, 1 bis 5 Stunden lang bei 150°C bis 220°C erhitzt, das Pyrodextrin in 30 bis 50 Gewichtsprozent Wasser, basierend auf dem Gewicht der Lösung, löst, die Lösung auf pH 5,5 bis 6,5 einstellt, sie mit 0,05 bis 0,2 Gewichtsprozent α-Amylase, basierend auf dem Pyrodextringewicht, 0,5 bis 2 Stunden lang bei 85°C bis 100°C behandelt, die Lösung mit 0,05 bis 0,2 Gewichtsprozent Glucoamylase, basierend auf dem Pyrodextringewicht, bei etwa 55°C 24 bis 48 Stunden lang behandelt, das hydrolysierte Pyrodextrin reinigt und das genannte Pyrodextrinhydrolysat mittels Chromatographie an Ionenaustauschharz abtrennt.

7. Verwendung nach Anspruch 6, worin das genannte Ionenaustauschharz ein Alkalimetall- oder Erdalkalimetalltyp eines stark sauren Ionenaustauschharzes ist.

## Revendications

1. Utilisation d'un hydrolysat de pyrodextrine raffinée pour la préparation d'un composé alimentaire destiné à réduire la sécrétion d'insuline sans affecter négativement/modifier la glycémie chez un animal, étant entendu que ledit hydrolysat de pyrodextrine raffinée peut être obtenu par chauffage d'amidon en présence d'un acide minéral et en présence d'eau en une quantité inférieure ou égale à environ 5 % par rapport au poids d'amidon pour préparer la pyrodextrine, hydrolyse de la pyrodextrine par une α-amylase et raffinage de la pyrodextrine hydrolysée.

2. Utilisation selon la revendication 1, étant entendu que ledit hydrolysat de pyrodextrine raffinée se compose structurellement de glucose présentant des liaisons 1→2 et 1→3 de même que des liaisons 1→4 et 1→6 et une partie de leurs groupements terminaux réducteurs provient de 1→6-anhydroglucose.

3. Utilisation selon la revendication 1, étant entendu que ledit hydrolysat de pyrodextrine raffinée est obtenu par chauffage dudit amidon en présence d'acide chlorhydrique en une quantité de 0,01 à 0,1 % en poids par rapport au poids dudit amidon à 150°C à 220°C pendant 1 à 5 h pour préparer la pyrodextrine, dissolution de la pyrodextrine dans de l'eau en une quantité de 30 à 50 % en poids par rapport au poids de la solution, ajustement du pH de la solution à 5,5 à 6,5, traitement de la solution par une α-amylase en une quantité de 0,05 à 0,2 % en poids par rapport au poids de pyrodextrine à 85°C à 100°C pendant 0,5 à 2 h et raffinage de la pyrodextrine hydrolysée.

4. Utilisation d'un hydrolysat de pyrodextrine raffinée pour la préparation d'un composé alimentaire destiné à réduire la sécrétion d'insuline sans affecter négativement/modifier la glycémie chez un animal, étant entendu que ledit hydrolysat de pyrodextrine raffinée peut être obtenu par chauffage d'amidon en présence d'un acide minéral et en présence d'eau en une quantité inférieure ou égale à environ 5 % par rapport au poids d'amidon pour préparer la pyrodextrine, hydrolyse de la pyrodextrine par une α-amylase, puis par une gluco-amylase, et soumission de la pyrodextrine hydrolysée à une chromatographie sur résine échangeuse d'ions pour raffiner la pyrodextrine hydrolysée.

5. Utilisation selon la revendication 4, étant entendu que ledit hydrolysat de pyrodextrine raffinée se compose structurellement de glucose présentant des liaisons 1→2 et 1→3 de même que 1→4 et 1→6 et qu'une partie de leurs groupements terminaux réducteurs provient de 1→6-anhydroglucose.

6. Utilisation selon la revendication 4, étant entendu que ledit hydrolysat de pyrodextrine raffinée est obtenu par chauffage dudit amidon en présence d'acide chlorhydrique en une quantité de 0,01 à 0,1 % en poids par rapport au poids dudit amidon à 150°C à 220°C pendant 1 à 5 h pour préparer la pyrodextrine, dissolution de la pyrodextrine dans de l'eau en une quantité de 30 à 50 % en poids par rapport au poids de solution, ajustement du pH de la solution à 5,5 à 6,5, traitement de la solution par une α-amylase en une quantité de 0,05 à 0,2 % en poids par rapport au poids de pyrodextrine à 85°C à 100°C pendant 0,5 à 2 h, traitement de la solution par une gluco-amylase en une quantité de 0,05 à 0,2 % en poids par rapport au poids de pyrodextrine à environ 55°C pendant 24 à 48 h, raffinage de la pyrodextrine hydrolysée et soumission de la pyrodextrine hydrolysée et raffinée à une chromatographie sur résine échangeuse d'ions pour séparer ledit hydrolysat de pyrodextrine.

7. Utilisation selon la revendication 6, étant entendu que ladite résine échangeuse d'ions est un type à métal alcalin ou à métal alcalino-terreux de résine échangeuse d'ions fortement acides.
